# EUROPEAN PATENT APPLICATION

(11) **EP 2 249 154 A1**
(43) Date of publication of application: **10.11.2010**
(21) Application number: 09159634.6
(22) Date of filing: 07.05.2009
(51) Int. Cl.: G01N 33/50, G01N 33/68

(54) **Method for high recovery of serum low-abundance, low molecular-weight proteins and their analysis by matrix assisted laser desorption ionization mass spectrometry**

(71) Applicant: IRCCS San Raffaele Pisana- San Raffaele, 00166 Roma (IT)
(72) Inventor: Guadagni, Fiorella, 00163 Roma (IT); Di Girolamo, Francesco, 00163 Roma (IT)
(74) Representative: Capasso, Olga

(57) **Abstract**

It is described a method for the detection of LMW protein profile from a serum sample comprising the step of:
a) precipitating high abundant serum proteins and collecting supernatant;
b) purifying said collected supernatant with magnetic beads with reverse phase functional surfaces;
c) crystallizing on MALDI mass spectrometer plate the products of step b), using a MALDI crystallization technique, in order to obtain four crystallized homogeneous spots;
d) analyzing LMW protein profiles of all crystallized spots by means of Matrix-Assisted Laser Desorption Ionization/Time-of-Flight mass spectrometer (MALDI/ToF ).

## Description

### BACKGROUND

Early detection of many diseases can greatly improve living conditions of a patient. Unfortunately, only few specific biomarkers for diagnosis of early-stage disease [1], prognosis or non-responsiveness to therapy are available today [2].

Most of these molecules, used as biomarkers, suffer of poor sensitivity, specificity and predictive value, especially when applied to rare diseases and to neurodegenerative, cardiovascular and oncologic diseases [3].

In order to obtain highly sensitive and specific biomarkers, it is essential to:
1) work on biological samples that should be as representative as possible of patient's physiopathological condition;
2) employ analytical methodologies capable to identify and to rigorously validate molecular indicators - biomarkers.

As regards point 1, both, plasma and serum, have been used for decades as a source of proteins and other agents indicating changes of the physiological state of an individual (molecular biomarkers) [4]. The currently known components of the serum proteome are only the tip of the iceberg: they potentially contain elements of all proteins produced in the body [5]. Human serum proteome consists of a large variety of proteins with different chemical and physical properties present in sera at different concentrations [6].

The complexity of serum makes it a very informative source for developing a proteomic pattern. Biomarkers are often low abundance LMW secreted into the bloodstream as a result of the disease process. [8].

Serum contains about 10,000 different proteins, the majority of which are LMW protein species [7] and a total of 60-80 mg/mL of proteins, with a concentration range spanning at least nine orders of magnitude. LMW proteins are present in the serum in two forms: free and linked to carrier proteins, such as albumin and immunoglobulins (IgG), 65-97% of all serum proteins. As regards point 2, it is essential to use a highly specific and sensible analytical methodology to collect and identify serum LMW proteins as well as to obtain highly reproducible serum profiles to highlight the tiny differences between patients (independently from pathology) and control subjects and among specific diseases.

This idea has not yet materialized due to the many problems in standardizing the protocol at each analytical stage (pre-analytical, analytical and post-analytical) and to the extreme lability of the serum proteome [7a]. In addition, commercial kits developed so far by simply making a targeted depletion of the most abundant proteins (albumin and IgG) are not specific for the recovery of all LMW in serum.

Many potentially interesting biomarkers are linked to albumin and other highly abundant proteins in blood serum. For this reason Perkin Elmer developed PRO XPRESSION KIT to capture this carrier protein-bound LMW (the kit is now out of production). Many other potentially interesting LMW biomarkers are free (no-linked) and are extracted by means of profiling Kits (Invitrogen, Dynal Peptide Profiler and Bruker Daltonics Profiling kit).

Authors of the instant invention developed a method named: New Analytical Approach (NAA), to obtain a highly specific selective and reproducible protein profiling spectra of both LMW proteins, free and linked to abundance carrier proteins in the serum. Authors considered the many requirements due to the extreme lability of the serum proteome and standardized the experimental conditions of each analytical step, from blood sampling, to serum preparation and mass spectrometry analysis [10-12].

The authors of the invention set up a new analytical approach, allowing to obtain:
1) A high recovery of LMW proteins from serum, by means of specific solvents, as acetonitrile and trifluoroacetic acid (TFA) and homogeneous crystallization of sera proteins on a MALDI (Matrix Assisted Laser Desorption Ionization ) plate;
2) A high specific and reproducible serum LMW protein profiling, preferably by means of MALDI-TOF mass spectrometry technique.
   The method of the invention was compared with the method worked by the PRO XPRESSION KIT (PerkinElmer), Dynal peptide profiler (Invitrogen) and Profiling Kit (Bruker Daltonics) showing best reproducible results and best recovery of LMWs. MALDI/MS protein profiles as obtained by the two methods were divided in two classes and compared using the ClinProTools Software (Bruker Daltonics) for the statistic analysis of MALDI protein profiling [13]. Such analysis revealed that the method of the instant invention is able to recover a higher number of LMW proteins with respects to the other three kits.

Therefore, the specific object of the invention is a method for the detection of LMW protein profile from a serum sample comprising the step of:
a) precipitating high abundant serum proteins and collecting supernatant;
b) purifying said collected supernatant with magnetic beads with reverse phase functional surfaces;
c) crystallizing on MALDI mass spectrometer plate the products of step b), using a MALDI crystallization technique, in order to obtain four crystallized homogeneous spots;
d) analyzing LMW protein profiles of all crystallized spots by means of Matrix-Assisted Laser Desorption Ionization/Time-of-Flight mass spectrometer (MALDI/ToF ).

In a preferable embodiment the precipitation step of step a) is performed by means of acetonitrile/TFA precipitation.

In a preferable embodiment the purification step of step b) is performed by means of magnetic beads with C18 or C8 reverse phase functional surfaces, as i.e. RPC 18, Invitrogen or HIC-8, Bruker Daltonics.

It is also a specific object of the invention a kit to work the method as above disclosed comprising:
- Acetonitrile;
- Trifluoroacetic acid (TFA) ;
- Deionized water;
- α-cyano-4-hydroxycinnamic acid (CHCA) ;
- Magnetic beads with C18 or C8 reverse phase functional surfaces.

### Description of Figures

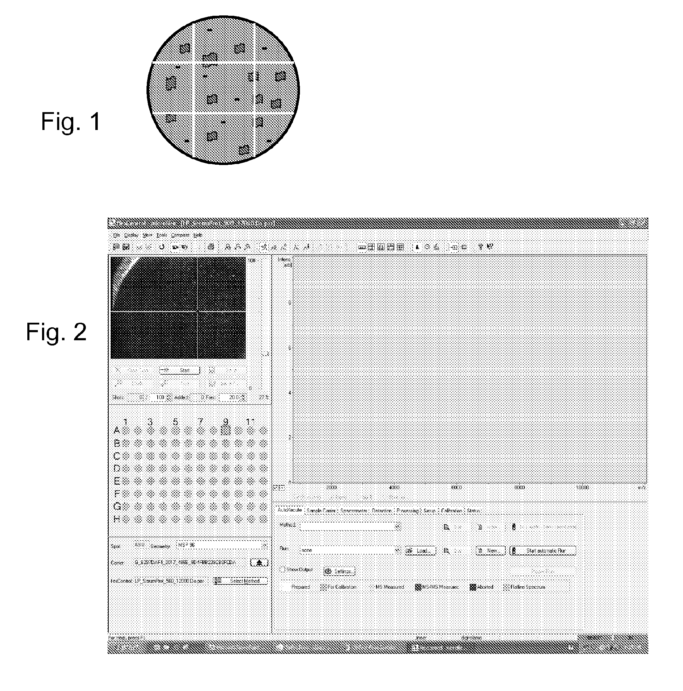

**Figure 1**. MALDI target's spots have been divided into nine equal areas and were 2 acquisitions of 100 shots each on good crystals of each area were performed.
**Figure 2**. Homogenous crystallization of serum sample over the spot MALDI (on the left of the picture): it allows to acquire very clean MS spectra and protein profiles easily comparable among them.

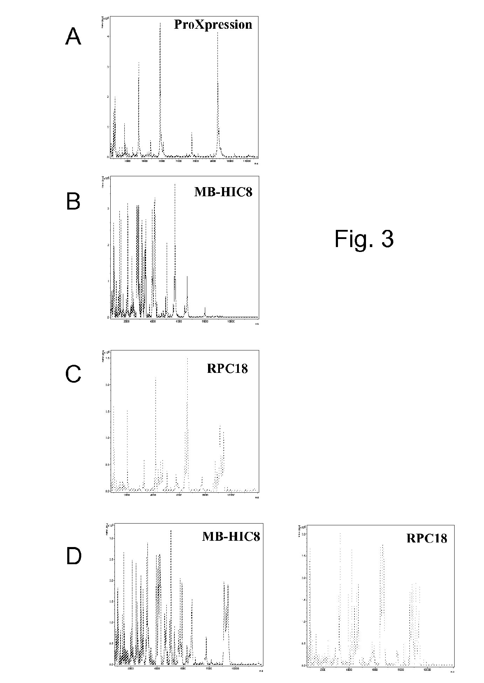

**Figure 3**. Low-Molecular-Weight Serum profiling from: A) ProXpression Kit Perkin Elmer): low number of LMW recovery (carrier protein-bound LMW) B) Profiling Kit MB-HIC8 (Bruker Daltonics): high number of LMW recovery (no-linked to albumin and other highly abundant proteins) C) Dynal Peptide Profiler RPC18 (Invitrogen): high number of LMW recovery (no-linked to albumin and other highly abundant proteins) D) NAA (reverse phase magnetic beads MB-HIC8 and RPC18 purification): best number of LMW recovery.

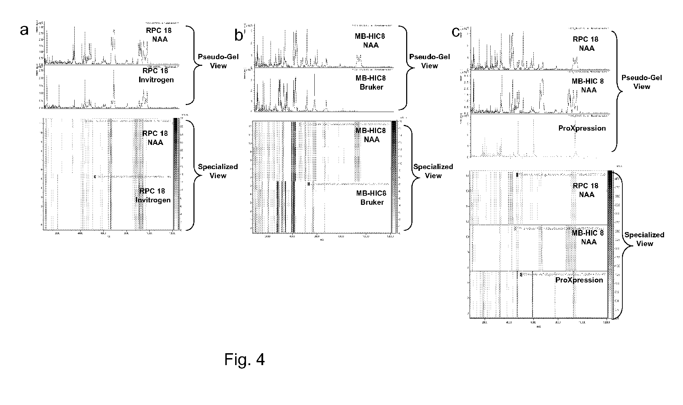

**Figure 4****.** Different viewer options in the ClinProTools bioinformatics package. The pseudo-gel view is the master navigation tool in a data set. All individual spectra are shown in a density scale. NAA MB-HIC8 contains 10 samples, NAA RPC 18 contains 10 samples, ProXpression Kit contains 10 samples: a) comparison between spectra acquired by Dynal Peptide Profiler RPC18 and NAA RPC 18 protocols, b) comparison between spectra acquired by Profiling Kit MB-HIC8 and NAA RPC 18 protocols, c) comparison between spectra acquired by ProXpression Kit and NAA RPC 18 and MB-HIC8 protocols. All spectra were acquired into the region of 0-12,000 Da with multiple differentially displayed signals.

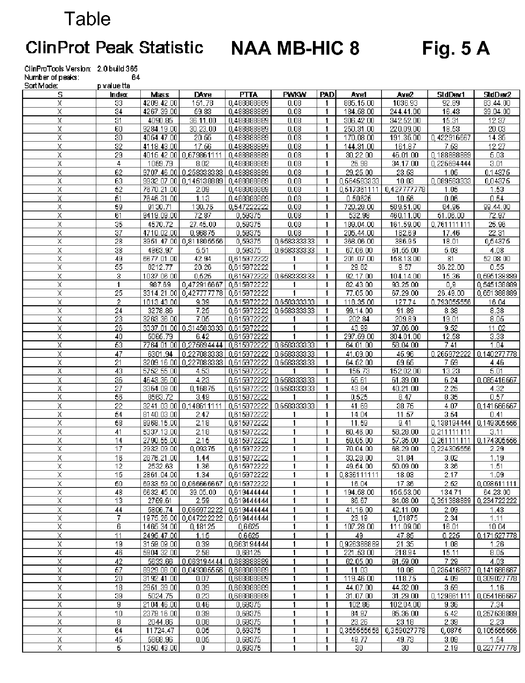

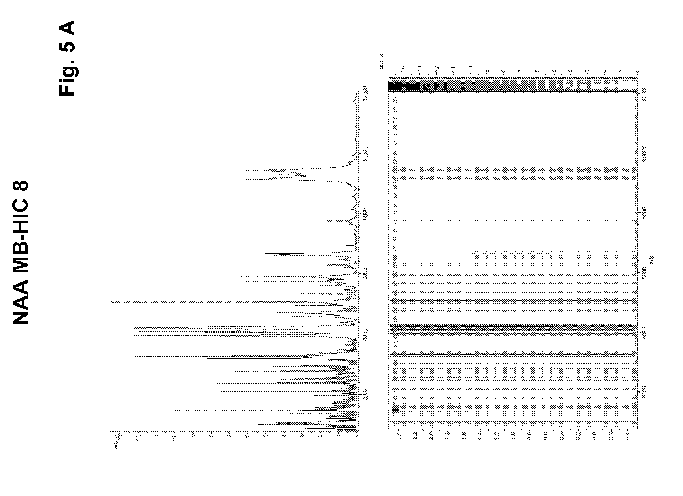

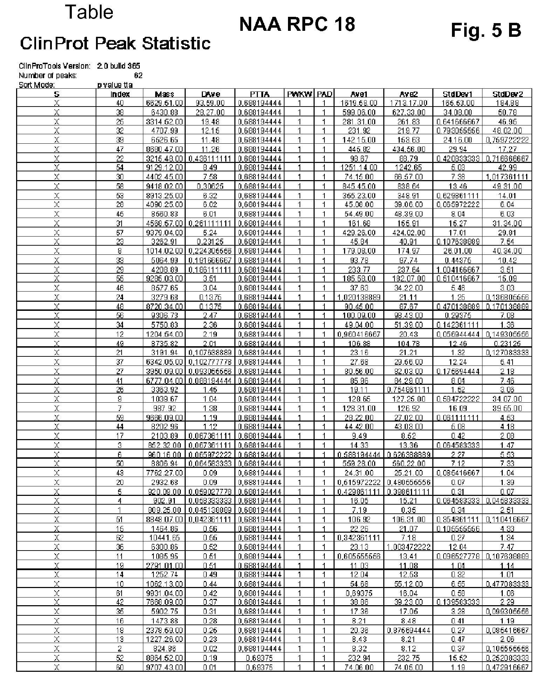

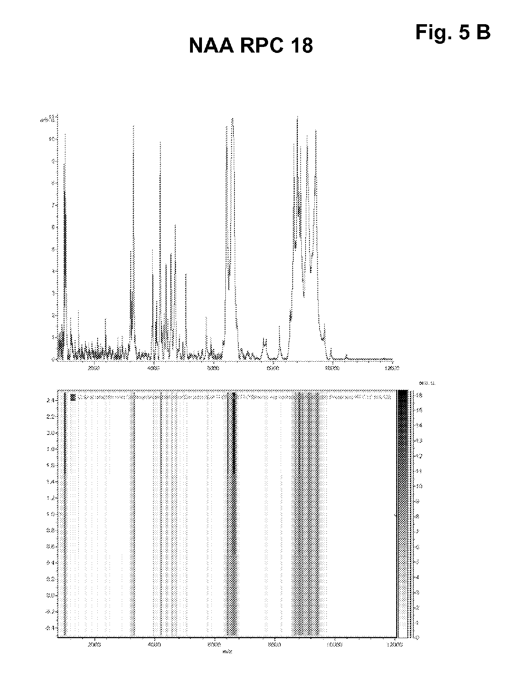

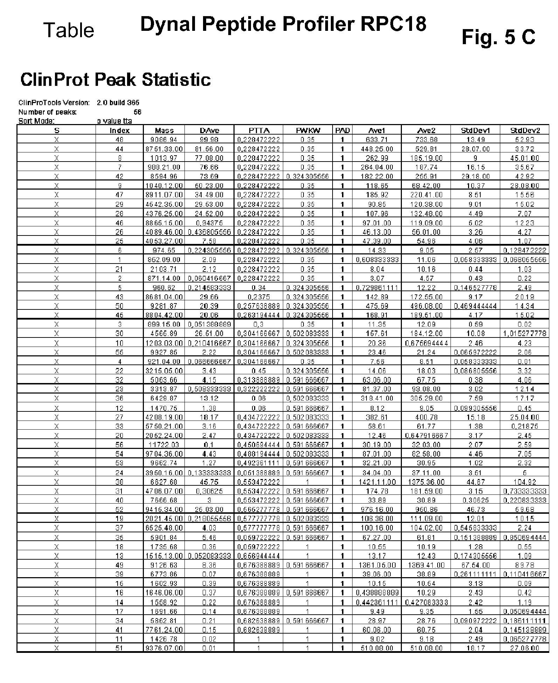

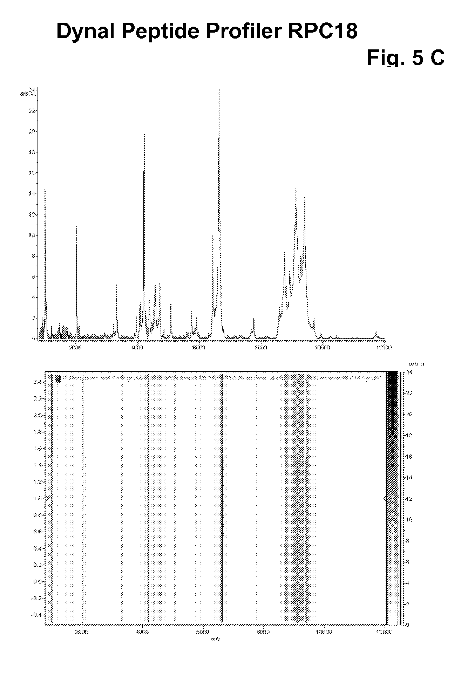

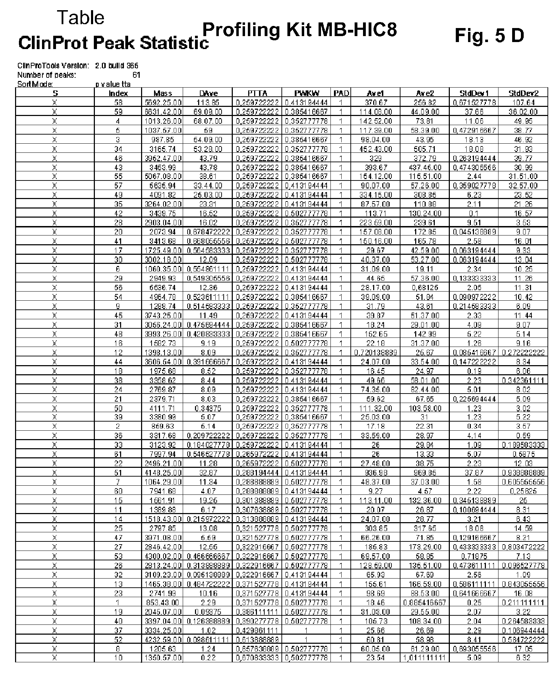

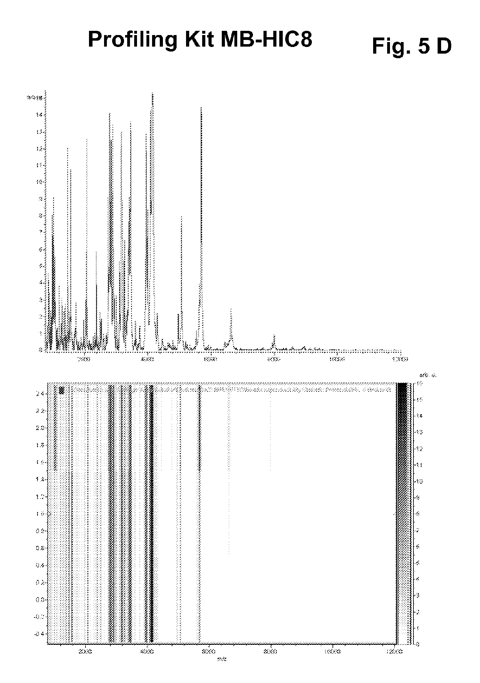

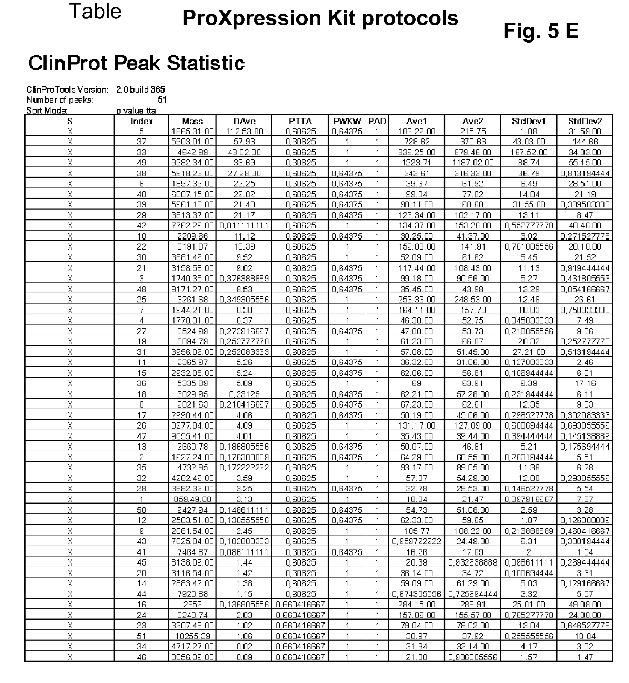

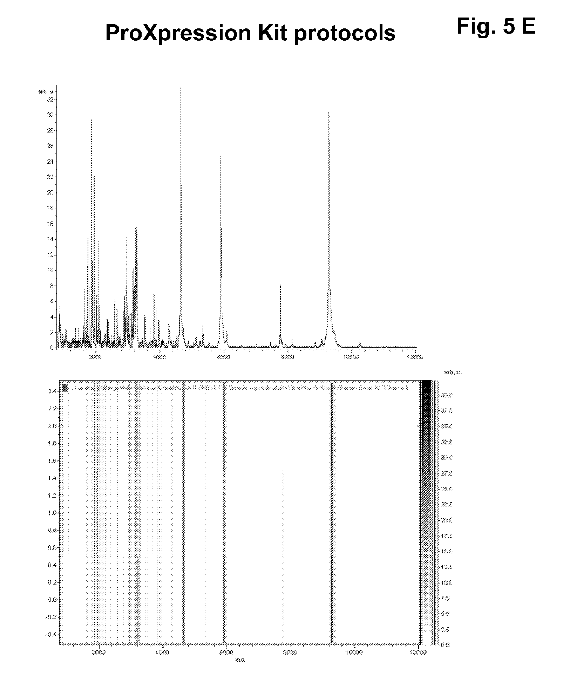

**Figure 5****. Feedback between visualization and peak statistics.** A,B,C, D and E Tables show all peaks statistics results calculated with the ClinProTools software. The quality of the peaks and the reproducible value of the analytical protocols can be directly evaluated in the visualization tools. NAA (MB-HIC 8: panel A and RPC 18: panel B) can identify more LMW proteins than Dynal Peptide Profiler RPC18 (panel C) Profiling Kit MB-HIC8 (panel D) and ProXpression Kit protocols (panel E). Moreover, the statistics of NAA is better than ProXpression Kit.

### EXPERIMENTAL

### Chemical

The α-Cyano-4-hydroxycinnamic acid (CHCA), ProteoMass Insulin MALDI/MS Standard, Bombesin protein standard and Trifluoroacetic acid (TFA) were purchased from Sigma-Aldrich (St. Louis, MO, USA), D-PBS (Dulbecco's Phosphate-Buffered Saline) was purchased from Invitrogen (Madison, Wisconsin 53719, USA), Hyper grade for liquid chromatography (LC/MS) Acetonitrile and Water for chromatography were acquired from Merk (Darmstadt, Germany), Complete EDTA-free Protease Inhibitor Cocktail Tablets from Roche (Mannheim Germany), BCA Protein Assay Kit from Thermo Scientific (N. Meridian Rd., Rockford USA), Magnetic Beads MB-HIC8 were obtained from Bruker Daltonics (Leipzig, Germany), Magnetic Beads RPC 18 were obtained from Invitrogen (USA), and ProXpression Kiti from PerkiElmer (Boston, USA).

### Method

### Serum Collection

Blood samples were obtained from five healthy volunteers (3 men and 2 women from 20 to 40 years) and collected into 4 mL Vacutanier tubes (Becton Dickinson, Franklin Lakes, NJ, USA) without anticoagulant and containing protease inhibitors. After about 30 minutes, serum was prepared by centrifugation at 3000 rpm for 10 min. Fresh serum was added to form a single sample of serum, aliquoted to 200 uL and immediately stored at - 80°C.

### Purification through NAA and MALDI target spotting of low-abundance LMW

All reagents must be freshly prepared.

Two aliquots of 80 µL serum were treated with 160 µL Acetonitrile/TFA 0.1 % solution, shacked vigorously and allowed to rest for 30 minutes at 4°C. Precipitated serum samples were centrifuged (10000g for 5 min at 4°C). The pellet was discarded and the supernatant was concentrated to 80 µL in a Speed Vac (Thermo Savant). Protein amount was assessed using the MicroBCA assay using BSA as a standard (Pierce, Rockford, IL), according to the manufacturer's instructions. The sample (5 µL, about 2.5 µg), was twice purified using magnetic beads with reverse phase functional surfaces (RPC 18 Invitrogen and MB-HIC-8 Bruker Daltonics) to generate a biomarker pattern for the profiling; the preparation was performed according to the manufacturer's instructions.

Each purified sample (1 µL) was deposited twice on two MALDI target's spots. The sample's spots were dried to air (from 3 to 10 minutes), and crystallized with 1 uL fresh alpha -ciano-4-hydroxycinnamic acid (3 mg/mL in 50% ACN/2% TFA).

Following this initial step the authors obtained four MALDI target's spots from two samples (loaded in duplicate) obtained after two purifications of a serum sample with MB-HIC8 and four MALDI target's spots from two samples (loaded in duplicate) obtained after two purifications of a serum sample with RPC 18.

*Purification through Dynal Peptide Profiler RPC18 (Invitrogen), [14] Profiling Kit MB-HIC8 (Bruker Daltonics) [15] and ProXpression kit (Perkin Elmer) [16] and MALDI target spotting of low-abundance LMW.*

The same serum sample (the same amount of instant purification), was treated with Dynal Peptide Profiler RPC18 (Invitrogen), Profiling Kit MB-HIC8 (Bruker Daltonics) and Pro Xpression Kit (PerkinElmer) according to the manufacturer's instructions. Each sample was purified as above described, twice, and each purified sample (1 µL) was deposited twice on two MALDI target's spots. The sample's spots were dried to air (from 3 to 10 minutes), and crystallized with 1 uL of fresh alpha -ciano-4-hydroxycinnamic acid (3 mg/mL in 50% ACN/2% TFA). As a result, there were four MALDI target's spots from two samples (loaded in duplicate) from two purifications with Dynal Peptide Profiler RPC18 (Invitrogen), four MALDI target's spots from two samples (loaded in duplicate) from two purifications with Profiling Kit MB-HIC8 (Bruker Daltonics) and four MALDI target's spots from two samples (loaded in duplicate) from two purifications with Pro Xpression Kit (PerkinElmer).

### Mass Spectrometry

Mass spectrometry was performed using the ClinProt system, equipped with an Microflex™ *linear* TOF instrument (Bruker Daltonik). Spectra have been collected automatically using the FlexControl™ software (Bruker Daltonik) for fuzzy-controlled adjustment of critical instrument settings to generate raw data of optimized quality.

To obtain the average spectra from NAA (MB-HIC8 and RPC18), from Profiling Kit MB-HICB, from Dynal Peptide Profiler RPC18 and from Pro Xpression Kit purification, the four spots were divided into nine equal areas (Figure 1) and 2 acquisitions of 100 shots on good crystals of each area were performed, for a total of 7,200 shots on the four sample spots resulting from the five different purifications. For an external calibration of the spectra, 1 pmol/uL of Bombesin (MW= 1619.85 Da) and Insulin (MW= 5729,61 Da) solution was used.

### Data Interpretation

The FlexAnalysis™ and ClinProTools™ softwares (Bruker Daltonik Bremen, Germany) were used for all data interpretation steps.

FlexAnalysis is a post-processing software for spectra acquired with the Bruker Time of Flight mass spectrometers of the flex series. FlexAnalysis works with highly sophisticated algorithms for authentic automated and interactive peak detection (SNAP, Centroid, and Sum Peak finder), for spectrum processing (smoothing, baseline substraction) and for automated and interactive recalibration of spectra (linear, quadratic, statistical and High Precision Calibration support). Though FlexAnalysis allows multiple spectrum display for convenient spectra comparison and analysis.

ClinProTools is an easy-to-use data post-processing software for visualization, data reduction, data mining, and building predictive models from protein profiling data using Bruker MALDI mass spectrometers (MS). ClinProTools combines intuitive visualization features and multiple mathematical algorithms to generate pattern recognition models for classification and prediction of e.g. disease from mass spectrometry based profiling data. These easy-to-use software features allows customers to rapidly generate and validate biomarker patterns from their protein profiling data.

### RESULTS AND CONCLUSIONS

The instant method (NAA) was developed and optimized to high recovery of LMW protein from serum and to obtain highly specific and reproducible serum LMW protein profiling through MALDI-TOF mass spectrometry technique.

The instant method (NAA) uses acetonitrile and trifluoroacetic acid precipitation, which has been shown to effectively precipitate large abundant proteins (albumin and immunoglobulins) out of the serum, to show up low abundance LMW peptides and proteins. Acetonitrile denatures high carrier proteins allowing carriers removal and causing the small, low-abundance proteins and peptides normally bound to these carrier proteins to dissociate. Funtionalized reverse phase magnetic beads, allow to recover these LMW thus making them available for detection by mass spectrometry [10, 11]. Profiling Kit MB-HIC8 and Dynal Peptide Profiler RPC18 are mixed with raw serum samples and allow to recovery only free LMW (no-linked to albumin and high abundant carrier proteins). The peptides and proteins extracted are subsequently eluted and analysed by mass spectrometry. The ProXpression Kit selectively captures abundant carrier proteins, followed by eluting and concentrating the biomarkers associated with them. This kit uses the Vivapure 96 Blue membranes that utilize the membrane chromatography technology of Vivascience AG combined with PerkinElmer's buffer and elution chemistries to quickly and reproducibly elute the peptide biomarkers bound to albumin. Both NAA sample, Profiling Kit MB-HIC8 sample, Dynal Peptide Profiler RPC18 sample and ProXpression Kit sample, were analyzed through mass spectrometry MALDI-TOF technique, therefore sample crystallization is crucial to obtain very clean spectra. Using sample crystallization technique for both experimental procedures above described, we obtained a very high sample crystallization homogeneous on MALDI target's spot (fig. 2) and very clean spectra easily comparable to each other. While ProXpression Kit allowed to recover only the LMW linked to high carrier protein, Profiling Kit MB-HIC8 and Dynal Peptide Profiler RPC18 both allowed to recover only the free LMW (no-linked with high carrier protein) in raw serum, the instant method retrieved both LMW-related carrier proteins and free LMW. Figure 3 shows the best number of LMW recovery of the instant method compared with the low number of LMW recovery of the Profiling Kit MB-HIC8, Dynal Peptide Profiler RPC18 and ProXpression Kit. Subsequently, ClinProTools software (Bruker Daltonik) was used for statistical analysis, data interpretation and comparison of MALDI-TOF spectra derived from the instant method, Profiling Kit MB-HIC8, Dynal Peptide Profiler RPC18 and Pro Xpression Kit LMW samples purification. ClinProTools offers a variety of viewer options for the analysis of clinical profiling data (Figure 4). A virtual gel view gives an overview representation of data sets derived from large sample cohorts. This viewer is the master navigation tool for the large data set. A representation of the signal intensities in a grey scale allows for detection of faint differences between the increasing peak height (Figure 4).

Finally, ten sample of each purification protocol (NAA HIC8: Fig. 5A, NAA RPC18: Fig. 5B, Dynal Peptide Profiler RPC18: Fig. 5C, Profiling Kit MB-HIC8: Fig. 5D, ProXpression Kit: Fig. 5E), were divided in two classes of five samples. These new classes were analyzed through ClinProTools software that provides a list of peaks sorted according to the statistical significance to differentiate between both classes. A list of all peaks sorted according to the statistical separation strength is created. Tables reported in Fig. 5, panels A-E show all peaks picked in peak calculation along with several values. The following data are displayed for each peak:
S, Inclusion/exclusion state of the peak: 'X' used for model generation (= included); '-' not used for model generation (= excluded)
*Index,* Peak index.
*Mass,* m/z value
*Dave,* Difference between the maximal and the minimal average peak area of all classes.
*PTTA,* P-value of t-test (2 classes) or ANOVA test (> 2 classes), range 0..1; 0: good, 1: bad. Preferable for normal distributed data.
*PWKW,* P-value of Wilcoxon (2 classes) or Kruskal-Wallis test (> 2 classes), range 0..1; 0: good, 1: bad. Preferable for not normal distributed data.
*PAD,* P-value of Anderson-Darling test; gives information about normal distribution; range 0..1; 0: normal distributed, 1: not normal distributed.
*AveN,* Peak area average of class N.
*StdDevN,* Standard deviation of the peak area average of class N.

### References

[1] Etzioni R, Urban N, Ramsey S, McIntosh M, Schwartz S, Reid B, Radich J, Anderson G, Hartwell L. The case for early detection. Nat Rev Cancer. 2003: 4, 243-52.
[2] Stoughton RB, Friend SH. How molecular profiling could revolutionize drug discovery. Nat Rev Drug Discov. 2005:4, 345-50.
[3] Eleftherios P. Diamandis, How Are We Going to Discover New Cancer Biomarkers? A Proteomic Approach for Bladder Cancer, Clinical Chemistry 2004: 5, 50
[4] Schrohl AS, Würtz S, Kohn E, Banks RE, Nielsen HJ, Sweep FC, Brünner N. Banking of biological fluids for studies of disease-associated protein biomarkers. Mol Cell Proteomics.2008: 2061-6.
[5] Lathrop JT, Hayes TK, Carrick K Hammond JD. Rarity gives a charm: evaluation of trace proteins in plasma and serum Expert Review of Proteomics Jun2005: 2, 393-406 .
[6] Petricoin EE, Paweletz CP, Liotta LA. Clinical applications of proteomics: proteomic pattern diagnostics. J mammary gland biol neoplasia 2002: 7, 433-440
[7] Joshua N. Adkins, Susan M. Varnum, Kenneth J. Auberry, Ronald J. Moore, Nicolas H. Angell, Richard D. Smith, David L. Springer, and Joel G. Pounds Toward a Human Blood Serum Proteome ANALYSIS BY MULTIDIMENSIONAL SEPARATION COUPLED WITH MASS SPECTROMETRY Molecular & Cellular Proteomics 2002: 12, 947-955
[7a] Lion N, Tissot JD Application of proteomics to haematology: the revolution is starting Expert Rev Proteomics 2008: 5, 375-379
[8] Anderson L, Anderson NG. The Human Plasma Proteome: History, Character, and Diagnostic Prospects 2002: 845-867
[9] Villanueva J, Shaffer DR, Philip J, Chaparro CA, Bromage HE, Olshen AB, Fleisher M, Lilja H, Brogi E, Boyd J, Carbayo MS, Holland EC, Cardo CC, Scher HI, Tempst P. Differential exoprotease activities confer tumor-specific serum peptidome patterns The Journal of Clinical Investigation 2006 116
[10] Albrethsen J. Reproducibility in protein profiling by MALDI-TOF mass spectrometry. Clin Chem. 2007:5, 852-8. Review.
[11] Merrell K, Southwick K, Steven W. Graves,a M. Sean Esplin,c Nathan E. Lewis,a and Craig D. Thulina,b, Analysis of Low-Abundance, Low-Molecular-Weight Serum Proteins Using Mass Spectrometry Journal of biomolecular techniques 2004: 15
[12] Chertov O, Biragyn A, Kwak LW, Simpson JT, Boronina T, Hoang VM, Prieto DA, Conrads TP, Veenstra TD, Fisher RJ. Organic solvent extraction of proteins and peptides from serum as an effective sample preparation for detection and identification of biomarkers by mass spectrometry. Proteomics 2004: 4, 1195-203.
[13] Ketterlinus R, Hsieh SY, Teng SH, Lee H, and Pusch W Fishing for biomarkers: analyzing mass spectrometry data with the new ClinProTools™ software BioTechniques. 2005: 37-40
[14] Villanueva J, Lawlor K, Toledo-Crow R , Tempst P Automated serum peptide profiling Nature Protocols. 2006: 1, 880-891
[15] M.E. de Noo, B.J. Mertens, A.Ozalp, M.R. Bladergroen, M.P.J. van der Werff, C.J.H. van de Velde, A.M. Deelder, R.A.E.M. Tollenaar. Detection of colorectal cancer using MALDI-ToF. serum protein profiling. European Journal of Cancer. 2006: 8, 1068-1076
[16] Lopez FM, Mikulskis A, Kuzdzal S, Bennett DA, Kelly J, Golenko E, DiCesare J, Denoyer E, Patton WF, Ediger R, Sapp L, Ziegert T, Lynch C, Kramer S, Whiteley GR, Wall MR, Mannion DP, della Cioppa G, Rakitan JS Wolf GM. High-Resolution Serum Proteomic Profiling of Alzheimer Disease Samples Reveals Disease-Specific, Carrier-Protein-Bound Mass Signatures. Clinical Chemistry 2005: 51, 1946-1954

## Claims

1. Method for the detection of LMW protein profile from a serum sample comprising the step of:
a) precipitating high abundant serum proteins and collecting supernatant;
b) purifying said collected supernatant with magnetic beads with reverse phase functional surfaces;
c) crystallizing on MALDI mass spectrometer plate the products of step b), using a MALDI crystallization technique, in order to obtain four crystallized homogeneous spots;
d) analyzing LMW protein profiles of all crystallized spots by means of Matrix-Assisted Laser Desorption Ionization/Time-of-Flight mass spectrometer (MALDI/ToF ).

2. Method for the detection of LMW protein profile from a serum sample according to claim 1 wherein the precipitation step of step a) is performed by means of acetonitrile/TFA precipitation.

3. Method for the detection of LMW protein profile from a serum sample according any of previous claims wherein the purification step of step b) is performed by means of magnetic beads with C18 or C8 reverse phase functional surfaces.

4. Kit to work the method for the detection of LMW protein profile according to any of previous claims comprising:
- Acetonitrile;
- Trifluoroacetic acid (TFA) ;
- Deionized water;
- α-cyano-4-hydroxycinnamic acid (CHCA) ;
- Magnetic beads with C18 or C8 reverse phase functional surfaces.
